# EUROPEAN PATENT SPECIFICATION

(11) **EP 3 865 572 B1**
(45) Date of publication and mention of the grant of the patent: **01.01.2025**
(21) Application number: 19870372.0
(22) Date of filing: 27.09.2019
(51) Int. Cl.: C12N 5/0797, C12N 5/02

(54) **MEDIUM FOR GROWTH OF NEURONAL STEM CELLS**
MEDIUM ZUM WACHSTUM VON NEURONALEN STAMMZELLEN
MILIEU POUR CROISSANCE DE CELLULES SOUCHES NEURONALES

(30) Priority: 11.10.2018 JP 2018192367
(43) Date of publication of application: 18.08.2021
(73) Proprietor: Ichimaru Pharcos Co., Ltd., Motosu-shi, Gifu 501-0475 (JP); Kobe Gakuin Educational Foundation, Kobe-shi, Hyogo 650-8586 (JP)
(72) Inventor: MASUTANI Teruaki, Motosu-shi, Gifu 501-0475 (JP); MIZUTANI Kenichi, Kobe-shi, Hyogo 650-8586 (JP)
(74) Representative: Hasegawa, Kan
(86) International application number: PCT/JP2019/038217
(87) International publication number: WO 2020/075534

(56) References cited:
- JP-A- 2002 069 097
- JP-A- 2005 218 308
- JP-A- 2005 278 641
- JP-A- 2007 124 982
- JP-A- 2010 529 855
- JP-A- H10 509 592
- JP-B2- S6 317 053
- US-A1- 2006 073 587
- US-A1- 2007 154 552
- US-A1- 2009 137 038
- OSTENFELD THOR ET AL: "Requirement for neurogenesis to proceed through the division of neuronal progenitors following differentiation of epidermal growth factor and fibroblast growth factor-2-responsive human neural stem cells", STEM CELLS,, vol. 22, no. 5, 1 January 2004 (2004-01-01), pages 798 - 811, XP002350600, ISSN: 1066-5099, DOI: 10.1634/STEMCELLS.22-5-798
- HIENOLA A ET AL: "HB-GAM inhibits proliferation and enhances differentation of neural stem cells", MOLECULAR AND CELLULAR NEUROSCIENCES, SAN DIEGO, US, vol. 26, no. 1, 1 May 2004 (2004-05-01), pages 75 - 88, XP008120809, ISSN: 1044-7431, [retrieved on 20040327], DOI: 10.1016/J.MCN.2004.01.018
- KLEINMAN ET AL: "Matrigel: Basement membrane matrix with biological activity", SEMINARS IN CANCER BIOLOGY, SAUNDERS SCIENTIFIC PUBLICATIONS, PHILADELPHIA, PA, US, vol. 15, no. 5, 1 October 2005 (2005-10-01), pages 378 - 386, XP005021947, ISSN: 1044-579X
- SAKAMOTO KAZUMA ET AL: "Glycan sulfation patterns define autophagy flux at axon tip via PTPR[sigma]-cortactin axis", NATURE CHEMICAL BIOLOGY, vol. 15, no. 7, 6 May 2019 (2019-05-06), New York, pages 699 - 709, XP093195109, ISSN: 1552-4450, Retrieved from the Internet <URL:http://www.nature.com/articles/s41589-019-0274-x> [retrieved on 20240814], DOI: 10.1038/s41589-019-0274-x
- KADOMATSU: "Proteoglycans and neuronal circuit reorganisation", OURNAL OF JAPANESE BIOCHEMICAL SOCIETY, vol. 83, no. 3, 1 January 2011 (2011-01-01), pages 240 - 246, XP093195117

## Description

### TECHNICAL FIELD

The present invention relates to a novel culture medium for the growth of neural stem cells, a method for the growth of neural stem cells using the culture medium, and the like.

### BACKGROUND ART

For the treatment of neurological diseases such as Parkinson's disease and spinal cord injury, transplantation of patient's own neural stem cells has been investigated because of shortage of donors and ethical problems (Non-Patent Document 1 and Patent Document 1).

The discovery of iPS cells has made it possible, for example, to create iPS cells from the patient himself and to induce neural stem cells from those iPS cells, and it is expected that autologous transplantation, in which one's own cells can be used for treatment, will be performed. However, it is believed to take a long time, more than six months, to create neural stem cells from a patient's own cells via iPS cells. In addition, the safety and direction of differentiation of iPS cells are thought to vary greatly from cell line to cell line, and it takes even longer to verify that the iPS cells are safe. Furthermore, in spinal cord injuries, it is believed that neural stem cells must be transplanted before the patient's symptoms are fixed in order to be effective. Therefore, for example, it is considered difficult to use the patient's own cells for treatment even with iPS cell transplantation technology in such diseases where the effective period of cell transplantation is limited (Non-patent document 1).

In addition, several methods for controlling differentiation of human neural cells from stem cells have been suggested (Non-Patent Document 2, Non-Patent Document 3, Patent Document 2).

In some studies, Matrigel^{®} is used as a biomatrix composition for supporting a wide variety of cell types (Non-Patent Document 4, Patent Document 3).

Patent Document 4 deals with the problem of providing a method for simply purifying cartilage type proteoglycan at a low cost without using a harmful reagent.

### PRIOR ART DOCUMENTS

### [Non-patent document]

[Non-Patent Document 1] Takeshi Matsui et al.:STEM CELLS 2012, 30:1109-1119
[Non-Patent Document 2] OSTENFELD THOR ET AL: "Requirement for neurogenesis to proceed through the division of neuronal progenitors following differentiation of epidermal growth factor and fibroblast growth factor-2-responsive human neural stem cells", STEM CELLS, vol. 22, no. 5, 1 January 2004 (2004-01-01), pages 798-811
[Non-Patent Document 3] HIENOLA A ET AL: "HB-GAM inhibits proliferation and enhances differentiation of neural stem cells", MOLECULAR AND CELLULAR NEUROSCIENCES, SAN DIEGO, US, vol. 26, no. 1, 1 May 2004 (2004-05-01), pages 75-88
[Non-Patent Document 4] KLEINMAN ET AL: "Matrigel: Basement membrane matrix with biological activity", SEMINARS IN CANCER BIOLOGY, SAUNDERS SCIENTIFIC PUBLICATIONS, PHILADELPHIA, PA, US, vol. 15, no. 5, 1 October 2005 (2005-10-01 ), pages 378-386

### [Patent Document]

[Patent Document 1] JP 2005-278641 A
[Patent Document 2] US 2006/073587 A
[Patent Document 3] US 2007/154552 A
[Patent Document 4] JP 2002-069097 A

### SUMMARY OF INVENTION

### PROBLEM TO BE SOLVED BY THE INVENTION

There is still a need, for example, for a means to produce neural stem cells (undifferentiated tissue stem cells of the nervous system) from patient's own cells quickly, easily, and safely, and more preferably without the inclusion of differentiated cells.

Therefore, the object of this invention is to provide a means to easily produce neural stem cells in a short period of time.

### MEANS FOR SOLVING THE PROBLEMS

The present invention has been made to solve such a problem, that is, in one aspect of the present invention, there is provided a culture medium for the growth of neural stem cells, the culture medium comprising FGF-2, EGF and proteoglycan. The proteoglycan is a chondroitin sulfate type proteoglycan, and the proteoglycan is a proteoglycan derived from salmon nasal cartilage.

In another embodiment, there is provided a method of growing neural stem cells, the method comprising the step of culturing neural stem cells using a culture medium comprising FGF-2, EGF, and proteoglycan.

### EFFECT OF THE INVENTION

By using this culture medium, neural stem cells can be easily produced in a short period of time.

### BRIEF DESCRIPTION OF THE DRAWINGS

[Fig.1] Fig.1 shows a schematic diagram of an experimental procedure performed in Experiment 1.
[Fig.2] Fig.2 shows the measurement result of the number of formed neurospheres in each treatment group. The vertical axis indicates the percentage(%) of formed neurospheres (NS), which specifically shows the ratio(%) of NS formed when the number of NS formed in the control group is 100(%). Statistical analysis using the Dunnet method was performed, and * indicates a significant difference, p < 0.05.
[Fig.3] Fig.3 shows the observations of neurospheres in each treatment group.
[Fig.4] Fig.4 shows a schematic diagram of an experimental procedure performed in Experiment 2.
[Fig.5] Fig.5 shows a schematic diagram of an experimental procedure performed in Experiment 3.
[Fig.6] Fig.6 shows the result of Experiment 4 (observation result of spheres). A bar, "-" in the figure shows 200µm.

### DESCRIPTION OF EMBODIMENTS

The following is a description of the culture medium of the invention.

### (Neural stem cells)

Neural stem cells are undifferentiated tissue stem cells of the nervous system that combine self-renewal and pluripotency. In the embryonic brain of humans and other mammals, neural stem cells first proliferate actively, increasing their number exponentially, and then generating asymmetric division. It is also known to produce astrocytes and oligodendrocytes in the late fetal and postnatal brain. Neural stem cells are the source of neurons, astrocytes, and oligodendrocytes, the major cell types that make up the central nervous system. In the present invention, preferably, cells from mammals such as humans, mice, and the like are used.

### (Proteoglycans)

Proteoglycans are glycoproteins with sugar chains called glycosaminoglycans such as chondroitin sulfate and dermatan sulfate (hereinafter referred to as GAGs) covalently attached to the core protein. Proteoglycans are widely distributed in the body, including skin and cartilage, as one of the major components of the extracellular matrix. GAG chains have a long linear structure with no branches. Because of the large number of sulfate and carboxyl groups, it is negatively charged, and the GAG chain takes on an extended shape due to its electrical repulsive force. In addition, proteoglycans can hold a large amount of water due to the water affinity of sugars. The large number of GAG chains in proteoglycans is responsible for the unique functions of cartilage, such as elasticity and resistance to impact, while flexibly holding water like a sponge.

The core protein of proteoglycans has the property of binding to various molecules in the matrix. In the case of cartilage proteoglycans, the N-terminus has a binding region for hyaluronan and link protein, which can bind to these substances, and also aggregate between the same molecules, and the C-terminus has lectin-like and EGF-like regions that can bind to various other molecules. These properties allow proteoglycans to develop structures that are unique to each tissue. Among proteoglycans, chondroitin sulfate-type proteoglycans are proteoglycans in which chondroitin sulfate is covalently bound to the core protein.

Salmon nasal cartilage-derived proteoglycans are proteoglycans obtained by extraction from salmon nasal cartilage. Here, the salmon is a fish belonging to the genus *Oncorhynchus,* for example, but the salmon with the scientific name *"Oncorhynchus keta"* is preferably selected from the viewpoint of cell proliferation (e.g., more efficient cultivation of the cells to be cultured). The lower limit of the proteoglycan content in the culture medium of the present invention, for example, in terms of cell proliferation, is preferably 1µg/ml or more, more preferably 2ug/ml or more, more preferably 5ug/ml or more, and even more preferably 10ug/ml or more. The proteoglycans contained in the culture medium according to the present invention are produced, for example, by the method described in Japanese Patent No. 6317053.

### (FGF-2)

FGF-2 (Fibroblast Growth Factor-2, also called bFGF) can be isolated from animal nervous tissue, pituitary gland, adrenal cortex, and placenta. FGF-2 is known to induce neural differentiation, survival, and regeneration, as well as regulate embryonic development and differentiation. FGF-2 has a wide range of functions, such as a cell growth factor, angiogenic factor, and neurotrophic factor, and shows proliferative activity against a variety of cells, including ES cells and iPS cells.

### (EGF)

EGF (Epidermal Growth Factor) is a polypeptide that promotes the proliferation of various cells including epithelial cells. EGF is not species specific and has proliferative effects on a variety of cells including epithelial cells, fibroblasts, and hepatocytes. In vivo, EGF is considered to be a growth factor that plays an important role in cell proliferation and differentiation.

### (Culture medium)

The basal culture medium used for supplementing FGF-2, EGF, and proteoglycans in the present invention can be any known culture medium. For example, a culture medium containing components necessary for cell survival and proliferation (inorganic salts, carbohydrates, hormones, essential amino acids, and vitamins) such as Iscove's modified Dulbecco's medium (IMDM), RPMI, DMEM **(e.g.,** DMEM/HamF-12 medium (Nacalai Tesque Co., Ltd., 08460-95) used in the following example), Fischer medium, α medium, Leibovitz medium, L-15 medium, NCTC medium, F-12 medium, MEM, McCoy medium) can be used. Further additives (e.g., antibiotics) may also be included as needed.

The culture medium used in the present invention may preferably not contain serum. The reason why serum may not be included is that serum contains unknown differentiation-inducing agents, which may inhibit differentiation of a high percentage of the neurons of the present invention. For example, if one wants to preferentially induce other than GABAergic neurons, the absence of serum is preferable because the presence of serum may increase the rate of induction of GABAergic neurons.

Although there is no particular limitation on the state of the substance of the culture medium used in the present invention, a liquid culture medium is preferred from the viewpoint of simplicity of culture medium preparation (e.g., medium preparation is considered to be easier when floating culture is performed).

### (Methods of Growing Neural Stem Cells)

Although there is no particular limitation on the period of time for culturing to grow neural stem cells using the culture medium of the present invention, it is preferable that the period of time is at least 3 days or more, preferably 5 days or more, and more preferably 7 days or more, after inoculating the cells to be cultured.

Although there is no particular limitation on the temperature to be cultured, from the viewpoint of further optimizing the growth, the lower limit is preferably 36 to 37°C or higher, more preferably 36°C or higher, still more preferably 36.5°C or higher, from the viewpoint of further optimizing the growth, the upper limit is preferably 38.5°C or less, more preferably 38°C or lower, and still more preferably 37.5°C or lower.

The CO₂ concentration of the environment in which the culture is conducted is not particularly limited, but from the viewpoint of further optimizing the growth, the lower limit is preferably 3.5% or more, more preferably 4% or more, and even more preferably 4.5% or more, and from the viewpoint of further optimizing the growth, the upper limit is preferably 10% or less, more preferably 6% or less, and even more preferably 5.5% or less. 5 % or less.

The method of this cultivation (e.g., floating culture, adhesive culture) is not limited, but floating culture is preferred due to its simplicity of cultivation (e.g., it is considered easier to homogeneously mix the contained components).

### EXAMPLES

### [Experiment 1: Measurement of the number of formed neurospheres (NS) and observation of NS]

To confirm that the cells cultured in the presence of FGF-2, EGF and proteoglycans are neural stem cells, the number of formed neurospheres (NS) were measured and the morphology of NS was observed.

### (Procedures for Culturing Neural Stem Cells)

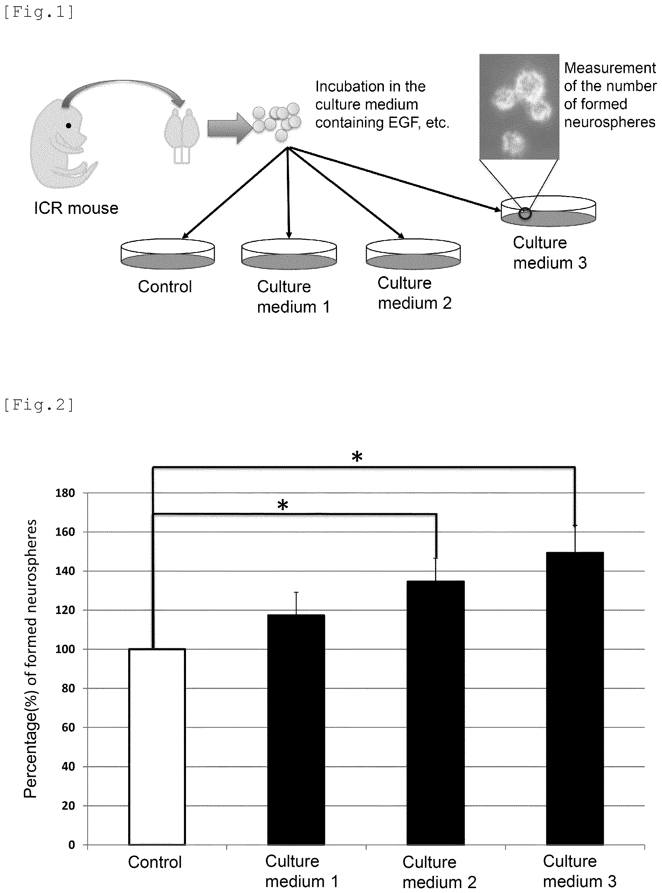

The procedure (experimental procedure) for culturing neural stem cells in Experiment 1 is described below. A schematic diagram of this experimental procedure is shown in Fig.1. A female mouse on the 14th day of gestation was prepared using ICR mice manufactured by Japan SLC Co. The fetus mouse present in the body of the female mouse was then removed, to prepare cells dispersed from the cerebral cortex of the fetus mouse. In this dispersion, 0.05w/v% trypsin (a product of Fujifilm Wako Pure Chemical Co., Ltd. (0.25w/v% trypsin-1mmol/l EDTA-4Na solution (containing phenol red) (product codes: 201-16945, 209-16941)) diluted in PBS) was used. The following treatment groups (control, culture medium 1, culture medium 2, and culture medium 3) were prepared using 240,000 cells of these dispersed cells. These cells were used 60,000 cells/each sample, respectively. The cells were inoculated into a 6-well plate (Thermo Fisher Scientific, Inc., CAT #140675) with the predetermined liquid culture medium. These cells were inoculated evenly 10,000 cells in each well of the 6-well plate with 2ml of the predetermined liquid culture medium. After 7 days of incubation, the formed cell aggregations in the 6-well plate of each treatment group were observed using a prescribed phase contrast microscope (magnification 20x). The cell aggregations with a radius of 50um or more were designated as neurospheres, and the number of neurospheres was measured. As a comparison to the observation after 7 days of incubation, the observation on the first day of incubation of each treatment group was also performed in the same way using a prescribed phase contrast microscope (magnification 20x).

The composition of the liquid culture medium for each dose group (control, culture medium 1, culture medium 2, and culture medium 3) is as follows. DMEM/HamF-12 (Nacalai Tesque Co., Ltd., 08460-95) was used as the basic medium in all dose groups, and the composition of each component in 50ml is shown below.

### (Control)

- N2 500µl
- B27 1ml
- Heparin 50µl (final concentration: 2ng/ml)
- Antibiotics 100µl (Final concentration of penicillin 100U/ml, streptomycin 10ug/ml)
- bFGF 50µl (final concentration 10ng/ml)
- EGF 50µl (final concentration 10ng/ml)
- No proteoglycan inclusion

### (Culture medium 1)

- N2 500µl
- B27 1ml
- Heparin 50µl (final concentration: 2ng/ml)
- Antibiotics 100µl (Final concentration of penicillin 100U/ml, streptomycin 10ug/ml)
- bFGF 50µl (final concentration 10ng/ml)
- EGF 50µl (final concentration 10ng/ml)
- Final proteoglycan concentration of 10ug/ml

### (Culture medium 2)

- N2 500µl
- B27 1ml
- Heparin 50µl (final concentration: 2ng/ml)
- Antibiotics 100µl (Final concentration of penicillin 100U/ml, streptomycin 10ug/ml)
- bFGF 50µl (final concentration 10ng/ml)
- EGF 50µl (final concentration 10ng/ml)
- Final proteoglycan concentration of 100ug/ml

### (Culture medium 3)

- N2 500µl
- B27 1ml
- Heparin 50µl (final concentration: 2ng/ml)
- Antibiotics 100µl (Final concentration of penicillin 100U/ml, streptomycin 10ug/ml)
- bFGF 50µl (final concentration 10ng/ml)
- EGF 50µl (final concentration 10ng/ml)
- Final proteoglycan concentration of 1000µg/ml

The ingredients contained in each of these dose groups (control, culture medium 1, culture medium 2, and example 3) were as follows.
- N2: N-2 Supplement(100X) (gibco:17502-048)
- B27: B-27^{™} Plus Supplement Vitamin-A-free (50X) (gibco:A35828-01)
- Heparin: Heparin sodium (Nacalai Tesque (product codes: 17513-96, 17513-41, 17513-54)
- Antibiotics: penicillin-streptomycin (WAKO: 168-23191)
- bFGF: bFGF(funakoshi:100-18B)
- EGF: EGF(funakoshi:315-09)
- Proteoglycan: Proteoglycan, derived from salmon nasal cartilage (Fujifilm Wako Pure Chemical Chemical Company (product code: 162-22131, 168-22133))

### (Results of Measurement of the Number of Formed Neurospheres (NS) in Each Treatment Group)

The results of this measurement are shown in Fig.2. What is shown in this figure is the percentage(%) of NS formation when the number of NS formation in the control group is 100(%). Culture medium 1 is 117.317753376014%, culture medium 2 is 134.677950940218%, and culture medium 3 is 149.305508553623%. It was observed that the number of NS formation increased with the increase in proteoglycan content. For culture medium 2 and culture medium 3, it was confirmed that the number of NS formation was increased with statistically significance compared to the control.

### (Results of NS observation)

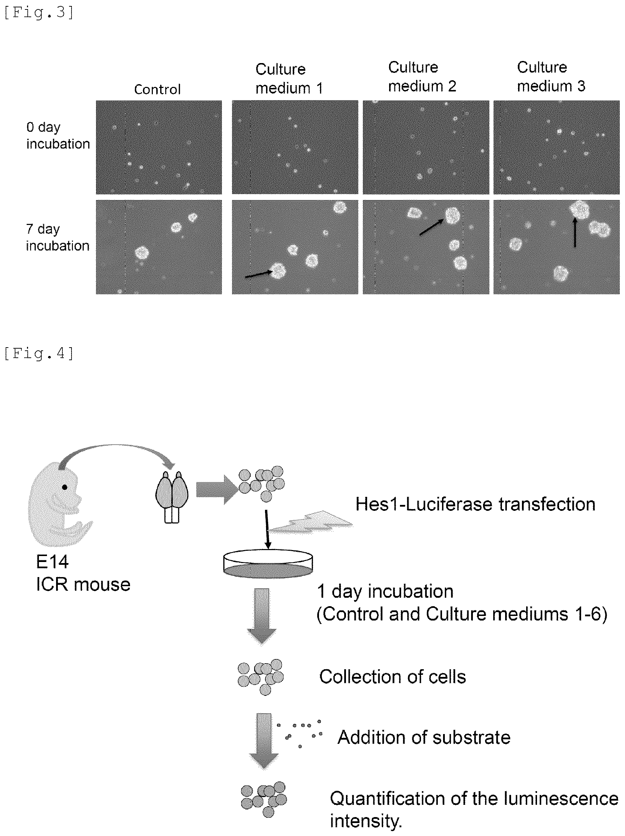

The results of this observation are shown in Fig. 3, where the observations after 7 days of incubation ("7 day incubation" in Fig. 3) are also compared with those after 0 days of incubation ("0 day incubation" in Fig. 3). Compared to the control, many more NS were observed in culture media 1-3. Some NSs are indicated by arrows in Fig. 3.

Therefore, it was found that much more neural stem cells proliferate by the inclusion of FGF-2, EGF and proteoglycans.

### [Experiment 2: Dual-luciferase assay (Confirmation of transcriptional activity of Hes-1)]

By checking the transcriptional activity of Hes-1, a protein (transcription factor) that regulates the maintenance of undifferentiated state of neural stem cells and their differentiation into astrocytes, we confirmed whether the neural stem cells described above (cells cultured in the presence of FGF-2, EGF, and proteoglycans) were maintained in their undifferentiated state.

The procedure for this dual-luciferase assay is described below. First, neural stem cells were prepared. A schematic diagram of this experimental procedure is shown in Fig. 4. We generated female mice on the 14th day of gestation using ICR mice manufactured by Japan SLC Co. The fetus mouse present in the body of the female mouse was then removed, to prepare cells dispersed from the cerebral cortex of the fetus mouse. In this dispersion, 0.05w/v% trypsin (a product of Fujifilm Wako Pure Chemical Co., Ltd. (0.25w/v% trypsin-1mmol/l EDTA-4Na solution (containing phenol red) (product codes: 201-16945, 209 -16941)) diluted in PBS) was used. The dispersed cells were divided into approximately 60,000 cells/each sample to prepare samples for the following seven treatment groups (control, culture medium 1, culture medium 2, culture medium 3, culture medium 4, culture medium 5, culture medium 6).

These samples were genetically transfected with Hes-1 cDNA and reporter gene (luciferase gene) using transfection reagent (Neon (registered trademark) Transfection System, Thermo Fisher Scientific). The Hes-1 cDNA and reporter gene (luciferase gene) were "pHes1(2.5k)-luc (Plasmid #43806)" manufactured by Addgene. After this gene transfer, the cells were cultured in the liquid culture medium of each treatment group (control, culture medium 1, culture medium 2, culture medium 3, culture medium 4, culture medium 5, and culture medium 6) at 37°C and 5% CO₂ for 1 day. After 1 day of culture, luciferase substrate (Thermo Fisher Scientific, Neon^{™} Transfection System) was added to each sample, and the expression of luciferase in each sample was observed using a detection system (Promega Corporation, GloMax^{™} Discover Microplate Reader).

The composition of the liquid culture medium for each dose group (control, culture medium 1, culture medium 2, culture medium 3, culture medium 4, culture medium 5, and culture medium 6) used in this dual-luciferase assay is as follows. The composition of the control, culture medium 1, culture medium 2, and culture medium 3 is the same as the liquid culture medium used in Experiment 1 above. DMEM/HamF-12 (Nacalai Tesque Co., Ltd., 08460-95) was used as the basic medium in all dose groups, and the composition of each component in 50ml is shown below. In this culture, "Costar^{™} 24-well" from Corning Inc. was used.

### (Control)

- N2 500µl
- B27 1ml
- Heparin 50µl (final concentration 2ng/ml)
- Antibiotics 100µl (Final concentration of penicillin 100U/ml, streptomycin 10ug/ml)
- bFGF 50µl (final concentration 10ng/ml)
- EGF 50µl (final concentration 10ng/ml)
- No proteoglycan is included.

### (Culture medium 1)

- N2 500µl
- B27 1ml
- Heparin 50µl (final concentration 2ng/ml)
- Antibiotics 100µl (Final concentration of penicillin 100U/ml, streptomycin 10ug/ml)
- bFGF 50µl (final concentration 10ng/ml)
- EGF 50µl (final concentration 10ng/ml)
- Final concentration of proteoglycan 10ug/ml

### (Culture medium 2)

- N2 500µl
- B27 1ml
- Heparin 50µl (final concentration: 2ng/ml)
- Antibiotics 100µl (Final concentration of penicillin 100U/ml, streptomycin 10ug/ml)
- bFGF 50µl (final concentration 10ng/ml)
- EGF 50µl (final concentration 10ng/ml)
- Final proteoglycan concentration of 100ug/ml

### (Culture medium 3)

- N2 500µl
- B27 1ml
- Heparin 50µl (final concentration: 2ng/ml)
- Antibiotics 100µl (Final concentration of penicillin 100U/ml, streptomycin 10ug/ml)
- bFGF 50µl (final concentration 10ng/ml)
- EGF 50µl (final concentration 10ng/ml)
- Final proteoglycan concentration of 1000µg/ml

### (Culture medium 4)

- N2 500µl
- B27 1ml
- Heparin 50µl (final concentration: 2ng/ml)
- Antibiotics 100µl (Final concentration of penicillin 100U/ml, streptomycin 10ug/ml)
- No bFGF inclusion
- EGF 50µl (final concentration 10ng/ml)
- Final proteoglycan concentration of 1000µg/ml

### (Culture medium 5)

- N2 500µl
- B27 1ml
- Heparin 50µl (final concentration: 2ng/ml)
- Antibiotics 100µl (Final concentration of penicillin 100U/ml, streptomycin 10ug/ml)
- bFGF 50µl (final concentration 10ng/ml)
- No EGF inclusion
- Final proteoglycan concentration of 1000µg/ml

### (Culture medium 6)

- N2 500µl
- B27 1ml
- Heparin 50µl (final concentration: 2ng/ml)
- Antibiotics 100µl (Final concentration of penicillin 100U/ml, streptomycin 10ug/ml)
- bFGF 150µl (final concentration 30ng/ml)
- EGF 50µl (final concentration 10ng/ml)
- No proteoglycan inclusion

The ingredients contained in each of these treatment groups (control, culture medium 1, culture medium 2, culture medium 3) were as follows.
N2: N-2 Supplement (100X) (Gibco: 17502-048)
B27: B-27^{™} Plus Supplement containing no vitamin A (50X) (gibco: A35828-01)
Heparin: Heparin sodium (Nacalai Tesque (product codes: 17513-96, 17513-41, 17513-54))
Antibiotics: penicillin-streptomycin (WAKO: 168-23191)
Antibiotics: Penicillin-streptomycin (WAKO: 168-23191)
bFGF: bFGF (funakoshi: 100-18B)
EGF: EGF (funakoshi: 315-09)
Proteoglycan: Proteoglycan, derived from salmon nasal cartilage (FUJIFILM Wako Pure Chemicals Company (product codes: 162-22131, 168-22133))

The observation of the expression level of luciferase (transcriptional activity of Hes-1) in each well is described below. When the expression level of the control group was 100, the expression level of the group in culture medium 1 was 123.83, the expression level of the group in culture medium 2 was 131.24, the expression level of the group in culture medium 3 was 159.51, the expression level of the group in culture medium 4 was 113.85, the expression level of the group in culture medium 5 was 119.67, and the expression level of the group in culture medium 6 was 131.55. The higher the expression level of luciferase, the higher the transcriptional activity of Hes-1, and the more undifferentiated neural stem cells are maintained. Culture medium 6 is considered as a positive control, which maintains the undifferentiated potential of neural stem cells. However, compared to culture medium 6 (which does not contain proteoglycans but has 3-fold higher bFGF content compared to the control), culture medium 1, culture medium 2, and culture medium 3 groups (FGF-2 EGF and proteoglycans) maintained the undifferentiated nature of neural stem cells.

### [Experiment 3: Measurement of the number of NS formation]

In a culture medium containing GAGs instead of proteoglycans, we checked whether cells cultured in this culture medium could form NSs. In this experiment, GAGs, unlike proteoglycans, do not have a core protein bound to them.

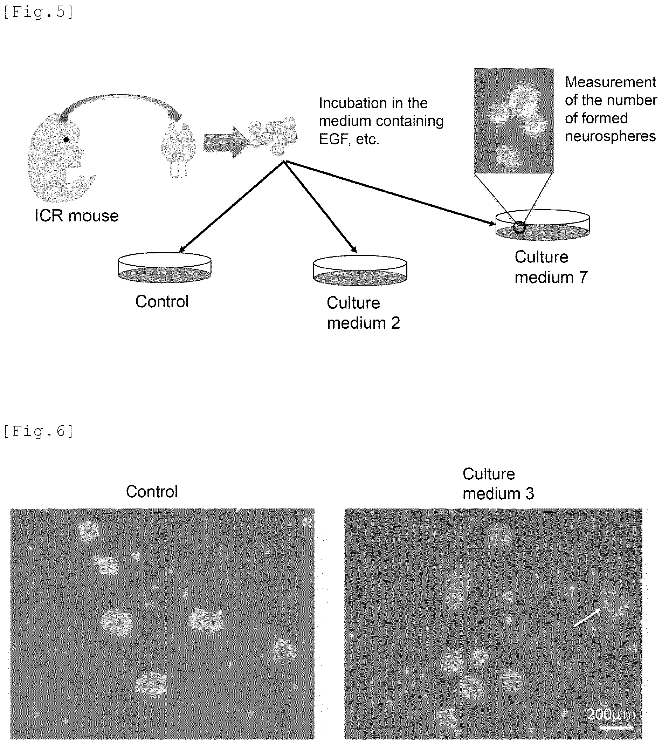

The procedure for this experiment 3 is described below. A schematic diagram of this experimental procedure is shown in Figure 5. A female mouse on the 14th day of gestation was created using ICR mice manufactured by Japan SLC Co. The mouse fetus present in the body of the mouse was then removed, and cells dispersed from the cerebral cortex of the mouse fetus were prepared. In this dispersion, 0.05w/v% trypsin (a product of Fujifilm Wako Pure Chemical Co., Ltd. (0.25w/v% trypsin-1mmol/l EDTA-4Na solution (containing phenol red) (product codes: 201-16945, 209 -16941)) diluted in PBS) was used. The following treatment groups (control, culture medium 2, culture medium 7) were prepared using 180,000 cells of these dispersed cells. These cells were used separately, 60,000 cells/each sample. The cells were inoculated into a 6-well plate (Thermo Fisher Scientific, Inc., CAT #140675) in which the prescribed liquid culture medium was present. 2ml of the prescribed liquid culture medium was inoculated evenly into each well of the 6-well plate so that 10,000 cells of these cells were present per well. After 6 days of incubation, the formed cell masses in the 6-well plate of each treatment group were observed under a prescribed phase contrast microscope (magnification 20x). After 6 days of incubation, the cell masses formed in the 6-well plates of each treatment group were observed under a phase contrast microscope (magnification: 20x), and the cell masses formed with a radius of 50um or greater were defined as neurospheres, and the number of these neurospheres was measured. As a comparison to the observation after 6 days of incubation, the observation on the first day of incubation of each treatment group was also performed in the same way using a prescribed phase contrast microscope (magnification 20x).

The composition of the liquid culture medium for each treatment group (control, culture medium 2, culture medium 7) is as follows. The composition of the control and culture medium 2 is the same as the liquid culture medium used in Experiment 1 above. DMEM/HamF-12 (Nacalai Tesque Co., Ltd., 08460-95) was used as the basic culture medium in all dose groups, and the composition of each component in 50 ml is shown below.

### (Control)

- N2 500µl
- B27 1ml
- Heparin 50µl (final concentration: 2ng/ml)
- Antibiotics 100µl (Final concentration of penicillin 100U/ml, streptomycin 10ug/ml)
- bFGF 50µl (final concentration 10ng/ml)
- EGF 50µl (final concentration 10ng/ml)
- No proteoglycan inclusion

### (Culture medium 2)

- N2 500µl
- B27 1ml
- Heparin 50µl (final concentration: 2ng/ml)
- Antibiotics 100µl (Final concentration of penicillin 100U/ml, streptomycin 10ug/ml)
- bFGF 50µl (final concentration 10ng/ml)
- EGF 50µl (final concentration 10ng/ml)
- Final proteoglycan concentration of 100ug/ml

### (Culture medium 7)

- N2 500µl
- B27 1ml
- Heparin 50µl (final concentration: 2ng/ml)
- Antibiotics 100µl (Final concentration of penicillin 100U/ml, streptomycin 10ug/ml)
- bFGF 50µl (final concentration 10ng/ml)
- EGF 50µl (final concentration 10ng/ml)
- Final GAG concentrations of 100ug/ml

The ingredients contained in each of these dose groups (control, culture medium 2, culture medium 7) were as follows.
- N2: N-2 Supplement(100X) (gibco:17502-048)
- B27: B-27^{™} Plus Supplement containing no vitamin A (50X) (gibco: A35828-01)
- Heparin: Heparin sodium (Nacalai Tesque (product codes: 17513-96, 17513-41, 17513-54))
- Antibiotics: penicillin-streptomycin (WAKO: 168-23191)
- bFGF:bFGF(funakoshi:100-18B)
- EGF: EGF(funakoshi:315-09)
- Proteoglycan: Proteoglycan, derived from salmon nasal cartilage (FUJIFILM Wako Pure Chemicals Company (product codes: 162-22131, 168-22133)), from which the GAG used in Experiment 3 is purified to be only GAG by the conventional method.

The results of the measurement of the number of formed NSs is described below. The number of formed NSs in the control group was 100(%), and the percentage of NSs formed in the control group was 58.8%. The number of NSs formed in the culture medium 2 group was 118.7%, and that in the culture medium 7 group was 58.8%. It was suggested that the structure of proteoglycans (the conjugate structure of the core protein and the GAG structure) promotes the formation of NS.

### [Experiment 4: Production results of stem cells that is capable of producing glia cells]

This section describes the possibility that the culture medium comprising FGF-2, EGF, and proteoglycan in the present invention can produce stem cells (aged cells compared to neural stem cells) that is capable of producing glial cells as well as neural stem cells. The experiment was conducted as follows.

First, neural stem cells were prepared as follows. A female mouse on the 14th day of gestation was created using ICR mice manufactured by Japan SLC Co. The mouse fetus in the body of the mouse was then removed, and cells dispersed from the cerebral cortex of the mouse fetus were prepared. In this dispersion, 0.05w/v% trypsin (a product of Fujifilm Wako Pure Chemical Co., Ltd. (0.25w/v% trypsin-1mmol/l EDTA-4Na solution (containing phenol red) (product codes: 201-16945, 209 -16941)) diluted in PBS) was used. The following dose groups (control, culture medium 3) were prepared using 120,000 cells of these dispersed cells. These cells were used separately, 60,000 cells/each sample. The cells were inoculated into a 6-well plate (Thermo Fisher Scientific, Inc., CAT #140675) in which the prescribed liquid culture medium was present. 2ml of the prescribed liquid culture medium was inoculated evenly into each well of the 6-well plate so that 10,000 cells of these cells were present per well. After this inoculation, the cells were cultured for 7 days at 37°C and 5% CO₂. After 7 days of culture, the formed cell aggregations were redispersed.

Then, the following dose groups (control, culture medium 3) were prepared using these redispersed cells (120,000 cells). These cells were used separately, 60,000 cells/each sample. The cells were inoculated in a 6-well plate (Thermo Fisher Scientific K.K., CAT #140675) in which the prescribed liquid culture medium was present. 2ml of the prescribed liquid culture medium was inoculated evenly in each well of the 6-well plate so that 10,000 cells of these cells were present per well. After the inoculation, the cells were cultured for 7 days at 37°C and 5% CO₂, and the number of formed spheres (secondary spheres) was checked using a phase contrast microscope (magnification 20x).

The control and culture medium 3 have the same composition as the liquid culture medium used in Experiment 1 above.

The experimental results are shown in Fig. 6. Fig. 6 shows a picture of this sphere (secondary sphere). The secondary spheres are indicated by the arrows in Fig. 6. In the group of culture medium 3, we could confirm the formation of secondary spheres (stem cells that is capable of producing glial cells). The number of secondary spheres formed in the culture medium 3 group was 132.7% of the number of formed spheres in the control group (100%).

## Claims

1. A culture medium for the growth of neural stem cells, comprising FGF-2, EGF and a proteoglycan, **characterized in that**
the proteoglycan is a chondroitin sulfate type proteoglycan, and
the proteoglycan is a proteoglycan derived from salmon nasal cartilage.

2. The culture medium according to claim 1 , wherein a content of the proteoglycan is 1µg/ml or more.

3. A use of a culture medium for the growth of neural stem cells, wherein the culture medium is according to claim 1 or 2.

## Patentansprüche

1. Kulturmedium für Wachstum neuraler Stammzellen, umfassend FGF-2, EGF und ein Proteoglycan, **dadurch gekennzeichnet, dass**
das Proteoglycan ein Proteoglycan vom Chondroitinsulfattyp ist und
das Proteoglycan ein Proteoglycan ist, das aus Nasenknorpel von Lachsen gewonnen wird.

2. Kulturmedium nach Anspruch 1, wobei der Gehalt an Proteoglycan-Gehalt 1 µg/ml oder mehr beträgt.

3. Verwendung eines Kulturmediums für Wachstum neuraler Stammzellen, wobei das Kulturmedium nach Anspruch 1 oder 2 ist.

## Revendications

1. Milieu de culture pour la croissance de cellules souches neurales, comprenant du FGF-2, de l'EGF et un protéoglycane, **caractérisé en ce que**
le protéoglycane est un protéoglycane de type sulfate de chondroïtine, et
le protéoglycane est un protéoglycane dérivé de cartilage nasal de saumon.

2. Milieu de culture selon la revendication 1 , dans lequel une teneur en protéoglycane est supérieure ou égale à 1µg/ml.

3. Utilisation d'un milieu de culture pour la croissance de cellules souches neurales, le milieu de culture se conformant à la revendication 1 ou 2.
